Europäisches Patentamt

European Patent Office

Office européen des brevets

(19)

(11) Numéro de publication : **0 414 590 B1**

(12) **FASCICULE DE BREVET EUROPEEN**

(45) Date de publication du fascicule du brevet :
**25.08.93 Bulletin 93/34**

(21) Numéro de dépôt : **90402273.8**

(22) Date de dépôt : **08.08.90**

(51) Int. Cl.$^5$ : **C10L 1/06,** C10G 29/20,
C07C 15/02

⸺

(54) **Procédé de reduction de la teneur en benzène des essences.**

(30) Priorité : **22.08.89 FR 8911199**

(43) Date de publication de la demande :
**27.02.91 Bulletin 91/09**

(45) Mention de la délivrance du brevet :
**25.08.93 Bulletin 93/34**

(84) Etats contractants désignés :
**BE DE FR GB IT NL**

(56) Documents cités :
**DE-A- 1 100 012**
**US-A- 2 396 682**
**US-A- 3 849 340**
**US-A- 4 140 622**

(73) Titulaire : **INSTITUT FRANCAIS DU PETROLE**
**4, Avenue de Bois Préau**
**F-92502 Rueil-Malmaison (FR)**

(72) Inventeur : **Collin, Jean-Claude**
**10, rue Agrippa d'Aubigné, Marsinval**
**F-78540 Vernouillet (FR)**
Inventeur : **Juguin, Bernard**
**46, avenue du Stade**
**F-92500 Rueil Malmaison (FR)**
Inventeur : **Larue, Joseph**
**97, Grande Rue**
**F-78240 Chambourcy (FR)**
Inventeur : **Rojey, Alexandre**
**52, rue Alexandre Dumas**
**F-92500 Rueil Malmaison (FR)**

EP 0 414 590 B1

## Description

La présente invention concerne un procédé de réduction de la teneur en benzène des essences. En effet, les législateurs mondiaux et principalement européens vont imposer pour des raisons de santé publique la limitation voire l'annulation de la teneur en benzène des essences hydrocarbonées et en particulier des réformats (réformats catalytiques).

Le procédé selon l'invention permet de diminuer voire annuler la teneur en benzène des essences en alkylant le benzène contenu dans l'essence par des oléfines extraites d'un gaz. Il a déjà été envisagé d'alkyler directement le benzène par un gaz contenant des oléfines en envoyant dans le réacteur d'alkylation les essences mélangées à la totalité du gaz.

Ainsi le brevet US-A-4.140.622, les essences sont fractionnées dans une première étape, la fraction légère comprenant pratiquement la totalité du benzène est additionnée d'un gaz chargé en oléfines, puis envoyée dans un réacteur d'alkylation, l'alkylat est fractionné et une fraction lourde pratiquement dépourvue de benzène est récupérée.

Ce système présente les inconvénients suivants :
- une dilution des réactifs ce qui induit un accroissement de la taille des équipements et une perte importante de l'efficacité de la réaction d'alkylation,
- la pression d'alkylation étant classiquement comprise entre 2 et 10 MPa, il est nécessaire dans ces conditions de comprimer la totalité du gaz jusqu'au niveau de pression requis.

Le procédé de réduction de la teneur en benzène des essences hydrocarbonées selon l'invention permet d'éviter ces inconvénients.

Il comporte les étapes suivantes :

a) on fractionne au moins une essence hydrocarbonée en une fraction légère A enrichie en benzène et une fraction lourde B appauvrie en benzène,

b) on met en contact la fraction légère A avec un gaz contenant au moins une fraction d'oléfines dont le nombre d'atomes de carbone est compris entre 2 et 5 par molécule, ladite mise en contact étant effectuée à une température comprise entre -40°C et 10°C et à une pression comprise entre 0, 5 et 2MPa.

c) on sépare, à l'issue de l'étape d'absorption réfrigérée (b), un gaz résiduel appauvri en oléfines et une fraction liquide C enrichie en oléfines,

d) on soumet ladite fraction liquide C à une alkylation à une pression comprise entre 2 et 10MPa et avec un rapport molaire benzène/oléfine compris entre 0,5 et 2,

e) on fractionne le mélange issu de l'étape (d) en une phase gazeuse constituée majoritairement des gaz non transformés au cours de l'alkylation, et en une phase liquide contenant au moins en partie le benzène non alkylé et les alkylbenzènes,

f) on mélange la phase liquide issue de l'étape (e) avec la fraction lourde B issue de l'étape (a).

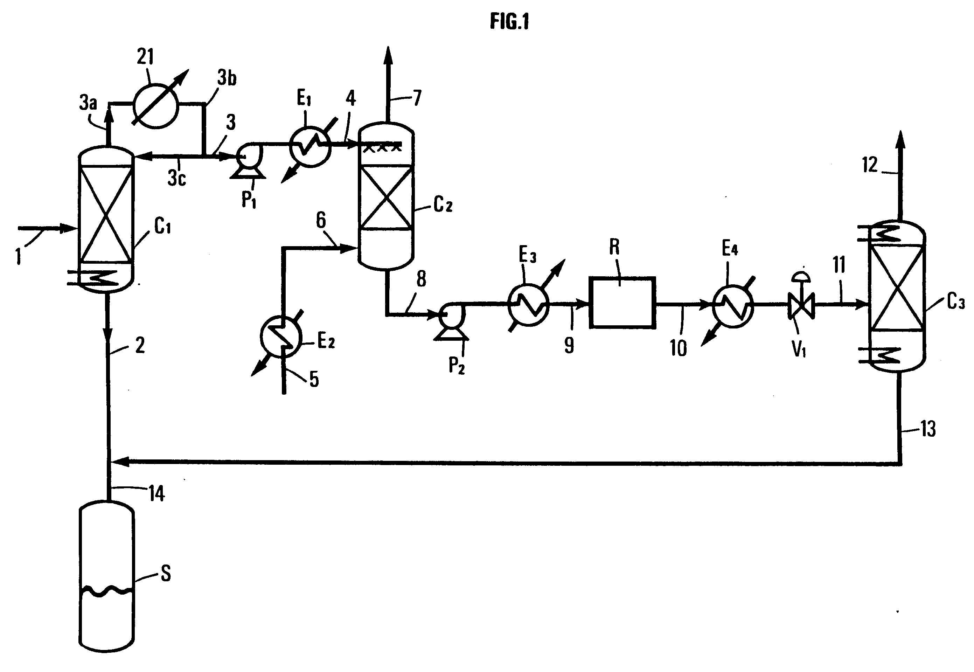

Il est illustré par le schéma de la figure 1. L'effluent de réformage contenant du benzène arrive par le conduit 1, il est fractionné dans la colonne de distillation C1 en deux parties :
- une fraction lourde B sortant en fond de la colonne C1 par le conduit 2, cette fraction B est appauvrie en benzène,
- une fraction légère A sortant en tête de la colonne C1 par le conduit 3, cette fraction A est enrichie en benzène. Les conditions opératoires de la colonne C1 seront choisies de manière que la fraction B contienne de préférence moins de 5 % en poids du benzène contenu dans l'effluent de réformage entrant dans la colonne C1.

La fraction légère A est pompée par la pompe P1, refroidie dans l'échangeur de chaleur E1 à une température inférieure à la température ambiante et entre par le conduit 4 dans la colonne d'absorption C2, dans laquelle elle est mise en contact avec un gaz contenant des oléfines qui arrive par le conduit 5 et est préalablement refroidi dans l'échangeur de chaleur E2 à une température inférieure à la température ambiante et proche de la température d'entrée de la fraction légère A et entre dans la colonne C2 par le conduit 6. Dans la colonne C2 il se produit une absorption d'une partie des oléfines du gaz dans la fraction légère A, le gaz appauvri en oléfines sort de la colonne C2 par le conduit 7. La fraction légère A ayant absorbé au moins une partie des oléfines du gaz sort de la colonne C2 par le conduit 8 et constitue la fraction liquide C. Ladite fraction liquide C est pompée par la pompe P2 à une pression supérieure, réchauffée dans l'échangeur E3 et entre par le conduit 9 dans le réacteur d'alkylation R.

Dans ce réacteur R, il se produit une réaction d'alkylation du benzène par les oléfines qui entraîne la formation d'alkylbenzènes. Le mélange sortant du réacteur R par le conduit 10 est refroidi dans l'échangeur de chaleur E4, détendu dans la vanne V1 jusqu'à une pression proche de la pression atmosphérique et entre dans la colonne de fractionnement C3 par le conduit 11.

Dans la colonne C3 on réalise une séparation, d'une part, des gaz non transformés qui sortent de la colonne C3 par le conduit 12 et, d'autre part, les autres constituants contenant en particulier les alkylbenzènes et le benzène non transformé ou résiduel lié à la réaction d'alkylation qui sortent de la colonne C3 en phase liquide par le conduit 13. Cette phase liquide est mélangée à la fraction lourde B issue de la colonne C1 et envoyée au stockage d'essence S sur la conduite 14.

En définitive le procédé selon l'invention est caractérisé en ce qu'il comprend les étapes suivantes :

a) on fractionne un effluent de réformage en une fraction légère A enrichie en benzène et une fraction lourde B appauvrie en benzène,

b) ladite fraction légère A est mise en contact à une température inférieure à la température ambiante avec un gaz contenant au moins une fraction d'oléfines dont le nombre d'atomes de carbone est compris entre 2 et 5 par molécule de manière à absorber dans ladite fraction légère A au moins une fraction desdites oléfines,

c) a l'issue de l'étape (b), on sépare un gaz résiduel appauvri en oléfines et une fraction liquide C enrichie en oléfines,

d) la fraction C issue de l'étape (c) est envoyée dans un réacteur d'alkylation de manière à alkyler au moins une fraction du benzène par au moins une fraction des oléfines,

e) le mélange issu de l'étape (d) est fractionné de manière à produire, d'une part, une phase gazeuse constituée majoritairement des gaz non transformés au cours de l'étape (d) et, d'autre part, d'une phase liquide contenant au moins en partie le benzène non alkylé et les alkylbenzènes,

f) la phase liquide issue de l'étape (e) est mélangée avec ladite fraction lourde B issue de l'étape (a).

La teneur en benzène de l'effluent de réformage peut varier en fonction de sa provenance, elle est en général inférieure à 10 % poids. Les conditions opératoires de la colonne C1 seront choisies de manière à ce que la fraction B contienne de préférence moins de 5 % poids du benzène contenu dans l'effluent de réformage entrant dans la colonne C1. En effet dans le but d'atteindre dans l'essence finale une teneur en benzène aussi faible que possible il y a avantage à opérer la séparation dans la colonne C1 de manière à minimiser la teneur en benzène de la fraction lourde B qui est envoyée directement dans les essences.

Le gaz circulant dans le conduit 5 peut provenir de différentes unités pétrochimiques, tel que par exemple craquage catalytique, vapocraquage... sa teneur en oléfines sera de préférence comprise entre 10 et 25 % poids. Ces oléfines sont majoritairement constituées d'éthylène, de propylène et d'une quantité moindre de butènes. Les autres constituants du gaz sont essentiellement des alcanes dont le nombre d'atome de carbone est compris entre 1 et 5 par molécule, de l'hydrogène et de l'azote. Il peut contenir en outre des proportions moindres d'autres gaz tel que dioxyde de carbone, monoxyde de carbone.

La pression de ce gaz est généralement comprise entre 0,5 et 2 MPa. Un des avantages du procédé selon l'invention est de pouvoir réaliser l'absorption des oléfines dans la fraction légère A sans avoir recours à une étape de recompression. En conséquence la pression de l'étape d'absorption (b) du procédé est comprise entre 0,5 et 2 MPa.

Dans les échangeurs E1 et E2, la fraction A, d'une part, et le gaz, d'autre part, sont refroidis de manière à favoriser l'absorption des oléfines du gaz dans la fraction liquide. Cette température de réfrigération est de préférence comprise entre 10 et -40 °C.

L'étape d'absorption (b) du procédé selon l'invention peut être réalisée selon différents modes. La colonne C2 peut être un contacteur à plateaux ou à garnissage. La technique la plus répandue est l'absorption adiabatique. Cependant l'absorption peut être réalisée de façon quasi isotherme par exemple soit dans une colonne à plateaux refroidie par des échangeurs intégrés sur les plateaux de la colonne, soit dans une colonne où la phase liquide de plusieurs plateaux est soutirée, refroidie dans un échangeur par un flux externe et réinjecté sur ledit plateau.

L'étape d'absorption peut être réalisée de façon isotherme par tout autre moyen connu de l'homme de l'art.

Dans l'étape d'alkylation (d), le rapport molaire benzène/oléfine doit être de préférence compris entre 0,5 et 2. Pour cela, les conditions opératoires de l'étape d'absorption (b) pourront être fixées de manière à satisfaire ce rapport pour le mélange entrant dans le réacteur d'alkylation. Cependant dans le cas où la quantité de benzène serait insuffisante, il pourra être procédé à un appoint de benzène dans la fraction A riche en benzène avant l'entrée dans le réacteur d'alkylation. Cet appoint peut provenir soit d'une unité extérieure au procédé selon l'invention, soit d'une partie de la fraction légère A circulant dans le conduit 3 auquel cas une partie est envoyée à la colonne d'absorption C2 et l'autre partie est mélangée aux produits circulant dans le conduit 8.

La réaction d'alkylation peut être effectuée par toutes les techniques connues de l'homme de l'art par exemple en utilisant un catalyseur. Ce catalyseur peut être constitué par exemple de mordénite ou de faujasite désaluminées.

Les conditions de pression de cette réaction sont en général comprises entre 2 et 10 MPa. Cette pression est en général supérieure à la pression à laquelle on peut disposer du gaz contenant les oléfines. Dans le cas du procédé selon l'invention le mélange entrant dans le réacteur est porté à la pression de la réaction par la pompe P2 sans qu'il soit nécessaire d'avoir recours à l'utilisation d'un compresseur.

L'absorption réfrigérée nécessite une source de réfrigération qui peut être fournie par un cycle de réfrigération externe dont la technique est connue de l'homme de l'art. Cependant dans le cas du procédé selon l'invention, il est possible de produire au moins une partie du froid nécessaire. Pour ce faire la phase gazeuse résiduelle circulant dans le conduit 7 issue de l'étape (c) est détendue dans une turbine de détente en produisant, d'une part, de l'énergie mécanique sur l'arbre de la turbine, et d'autre part, l'énergie frigorifique produite en réchauffant le gaz sortant de la turbine jusqu'à une température proche de la température ambiante.

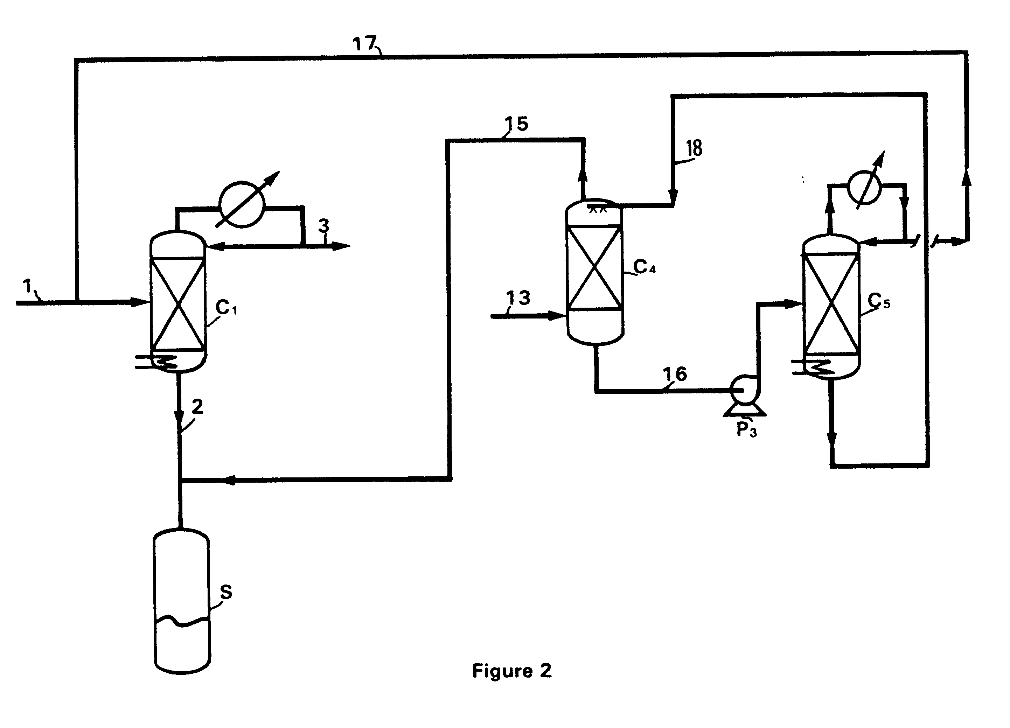

Le fait que la réaction d'alkylation nécessite un excès de benzène par rapport aux oléfines conduit à une teneur résiduelle en benzène dans les essences. Cette teneur résiduelle peut être pratiquement annulée dans une version plus élaborée du procédé qui est illustrée par le schéma de la figure 2.

La figure 2 représente la colonne de distillation C1, le bac de stockage S, et les équipements supplémentaires liés à la version élaborée du procédé. Les équipements compris entre les conduites 3 et 14 sont les mêmes que ceux de la figure 1. La phase liquide provenant de la colonne C3 formée en partie de la fraction légère A, additionnée des alkylbenzènes et du benzène résiduel est ici dirigée par le conduit 13 non pas vers le stockage d'essences mais d'abord dans une colonne C4. Cette colonne peut être un extracteur liquide-liquide ou une colonne à distiller permettant la réalisation d'une distillation extractive. Pour faciliter la compréhension de cette version élaborée du procédé, nous considérons que la colonne C4 est un extracteur liquide-liquide. Ladite phase liquide entrant dans l'extracteur C4, par le conduit 13, est mise en contact avec un solvant sélectif qui entre dans l'extracteur par le conduit 18. Dans l'extracteur C4, il se produit une extraction des composés aromatiques par le solvant ce qui conduit à :
- une phase raffinat sortant de la colonne C4 par le conduit 15, constituée en majorité par les constituants du mélange entrant par le conduit 13 appauvrie de la majorité des composés aromatiques. Ladite phase raffinat est mélangée à la fraction lourde B issue de la colonne C1 et envoyée au stockage d'essence S par la conduite 14.
- une phase extrait sortant de la colonne C4 par le conduit 16, constituée en majorité du solvant entrant par le conduit 18 enrichi des composés aromatiques extrait du raffinat.

Ladite phase extrait est pompée par la pompe P3 et injectée dans la colonne de distillation C5 afin de régénérer le solvant. Ledit solvant sort de la colonne C5 par le conduit 18 et recyclé vers l'extracteur C4. Les constituants aromatiques sortent de la colonne C5 par le conduit 17, ils sont mélangés à l'effluent de réformage entrant à l'étape (a) par le conduit 1.

Ainsi ledit effluent se trouve enrichi, d'une part, des alkylbenzènes et d'autre part, du benzène non transformé. Ils sont fractionnés dans la colonne C1, le benzène est entraîné dans la phase légère A (et est donc traité conformément à l'invention) les alkylbenzènes dans la fraction lourde B.

Dans cette version élaborée du procédé selon l'invention, les constituants aromatiques contenus dans le mélange liquide issu de l'étape (e) sont traités au cours d'une étape d'extraction par un solvant sélectif de manière à produire, d'une part, une phase raffinat exempte des constituants aromatiques et de solvant, cette dite phase raffinat est mélangée à la fraction lourde B issue de l'étape (a) et, d'autre part, une phase extrait contenant le solvant et les constituants aromatiques, cette dite phase extrait est fractionnée de manière à produire séparément le solvant qui est recyclé à l'étape d'extraction et les constituants aromatiques qui sont mélangés à l'effluent de réformage entrant à l'étape (a).

La phase d'extraction peut être réalisée soit en utilisant une extraction liquide-liquide, soit en pratiquant une distillation extractive. Le choix du solvant d'extraction est fait en fonction de la technique d'extraction retenue, il peut être choisi par exemple parmi les constituants suivants, ou un mélange de plusieurs d'entre eux ou un mélange de certaines d'entre eux avec l'eau : diméthylformamide, diméthylsulfoxyde, n-méthylpyrrolydone, anhydride sulfureux, sulfolane, diglycolamine, n-formylmorpholine, diéthylèneglycol, triéthylèneglycol, tétraéthylèneglycol.

Le principe du procédé selon l'invention est illustré par l'exemple 1.

## EXEMPLE

Dans cet exemple, on procède selon le schéma de la figure 1. L'effluent de réformage a une teneur en benzène de 5,91 % poids, sa densité à 20 °C est de 0,787, son débit est de 15.063 kg/h. Il entre par le conduit 1, dans une colonne de distillation C1 à plateaux où il est fractionné en deux parties :
- une fraction lourde B (85 °C point final) sortant en fond de la colonne C1 par le conduit 2, son débit est de 10.243 kg/h, sa teneur en benzène est inférieure à 0,05 % poids, sa densité à 20 °C est de 0,838.
- une fraction légère A (point initial 85 °C) sortant en tête de la colonne C1 par le conduit 3, son débit est de 4.820 kg/h, sa teneur en benzène est de 18,47 % poids, sa densité à 20 °C est de 0,687.

La fraction légère A est pompée par la pompe P1, refroidie dans l'échangeur de chaleur E1 à une température de -30 °C et entre par le conduit 4 à une pression de 1,5 MPa dans la colonne d'absorption C2, dans laquelle elle est mise en contact avec un gaz issu d'une unité de craquage catalytique.

Ce gaz a un débit de 997,2 kg/h, il est disponible à une pression de 1,55 MPa et de composition pondérale suivante :

- une fraction lourde B (85 °C point final) sortant en fond de la colonne C1 par le conduit 2, son débit est de 10.243 kg/h, sa teneur en benzène est inférieure à 0,05 % poids, sa densité à 20 °C est de 0,838.
- une fraction lourde B (85 °C point final) sortant en fond de la colonne C1 par le conduit 2, son débit est de 10.243 kg/h, sa teneur en benzène est inférieure à 0,05 % poids, sa densité à 20 °C est de 0,838.
- une fraction légère A (point initial 85 °C) sortant en tête de la colonne C1 par le conduit 3, son débit est de 4.820 kg/h, sa teneur en benzène est de 18,47 % poids, sa densité à 20 °C est de 0,687.

La fraction légère A est pompée par la pompe P1, refroidie dans l'échangeur de chaleur E1 à une température de -30 °C et entre par le conduit 4 à une pression de 1,5 MPa dans la colonne d'absorption C2, dans laquelle elle est mise en contact avec un gaz issu d'une unité de craquage catalytique.

Ce gaz a un débit de 997,2 kg/h, il est disponible à une pression de 1,55 MPa et de composition pondérale suivante :

| | |
|---|---|
| Hydrogène | : 0,46 |
| Monoxyde de carbone | : 4,47 |
| Dioxide de carbone | : 0,36 |
| Azote | : 15,03 |
| Oxygène | : 0,26 |
| Méthane | : 23,30 |
| Ethylène | : 16,41 |
| Ethane | : 23,62 |
| Propylène | : 5,85 |
| Propane | : 2,89 |
| Butanes | : 4,99 |
| Pentanes | : 2,26 |
| | ——— |
| | 100,00 % |

Ce gaz arrive par le conduit 5, est refroidi dans l'échangeur de chaleur E2 à une température de -30 °C et entre dans la colonne C2 par le conduit 6. Dans cette colonne C2 munie d'un garnissage il se produit une absorption d'une partie des oléfines du gaz dans la fraction légère A. Le gaz appauvri en oléfines sort de la colonne C2 par le conduit 7, à une température de -26 °C, il ne contient plus que 1,37 % d'oléfines, son débit est de 369 kg/h.

La fraction légère A ayant absorbé 97,5 % poids des oléfines du gaz, constitue la phase liquide C, qui sort de la colonne C2 à une température de -11 °C par le conduit 8, son débit est de 5.448 kg/h, sa composition donne un rapport molaire benzène/oléfines de 1,76 qui répond aux conditions requises pour la phase d'alkylation.

Ladite phase liquide C est pompée par la pompe P2 à une pression de 4,5 MPa et réchauffée dans l'échangeur E3 à une température de 250 °C et entre par le conduit 9 dans le réacteur d'alkylation R.

Dans ce réacteur R, il se produit une réaction d'alkylation du benzène par les oléfines qui entraîne une transformation de 90 % d'éthylène en éthylbenzène et une transformation de 100 % du propylène en propylbenzène. Le mélange sortant du réacteur R par le conduit 10 a un débit de 5.448 kg/h, il est refroidi dans l'échangeur de chaleur E4 à une température de 100 °C, détendu dans la vanne V1 à une pression de 0,15 MPa et entre dans la colonne de fractionnement C3 par le conduit 11.

Dans la colonne de fractionnement C3 on réalise la séparation du mélange en deux fractions :
1) une fraction gazeuse de 402 kg/h composés essentiellement des gaz non transformés qui sont évacués par le conduit 12, à une température de -10 °C.
2) une fraction liquide de 5.046 kg/h contenant en particulier les alkylbenzènes et le benzène résiduel lié à la réaction d'alkylation qui sortent de la colonne C3 à une température de 59.0 °C par le conduit 13.

Cette fraction liquide est mélangée au 10.243 kg/h de la fraction lourde B issue de la colonne C1 et envoyée au stockage d'essence S.

On obtient ainsi 15.289 kg/h d'essence soit une augmentation de rendement de 1,5 %. Cette augmentation est due aux constituants du gaz et plus particulièrement des oléfines qui s'absorbent dans la fraction légère A lors de la phase d'absorption.

La teneur de l'essence en benzène ainsi obtenue est réduite de 5,91 à 2,5 % poids soit une diminution de 57 % en poids.

## Revendications

1.  Procédé de réduction de la teneur en benzène des essences hydrocarbonées, caractérisé en ce que :
    a) on fractionne au moins une essence hydrocarbonée en une fraction légère A enrichie en benzène et une fraction lourde B appauvrie en benzène,
    b) on met en contact la fraction légère A avec un gaz contenant au moins une fraction d'oléfines dont le nombre d'atomes de carbone est compris entre 2 et 5 par molécule, ladite mise en contact étant effectuée à une température comprise entre -40°C et 10°C et à une pression comprise entre 0,5 et 2MPa.
    c) on sépare, à l'issue de l'étape d'absorption réfrigérée (b), un gaz résiduel appauvri en oléfines et une fraction liquide C enrichie en oléfines,
    d) on soumet ladite fraction liquide C à une alkylation à une pression comprise entre 2 et 10MPa et avec un rapport molaire benzène/oléfine compris entre 0,5 et 2,
    e) on fractionne le mélange issu de l'étape (d) en une phase gazeuse constituée majoritairement des gaz non transformés au cours de l'alkylation, et en une phase liquide contenant au moins en partie le benzène non alkylé et les alkylbenzènes,
    f) on mélange la phase liquide issue de l'étape (e) avec la fraction lourde B issue de l'étape (a).

2.  Procédé selon la revendication 1 caractérisé en ce que la fraction liquide C présente un rapport molaire benzène/oléfines compris entre 0,5 et 2.

3.  Procédé selon l'une des revendications 1 à 2 caractérisé en ce que l'étape d'absorption (b) est réalisée à une pression inférieure à la pression de l'étape d'alkylation (d).

4.  Procédé selon l'une des revendications 1 à 3 caractérisé en ce que l'étape d'alkylation (d) est réalisée avec un catalyseur type mordénite ou faujasite désaluminées.

5.  Procédé selon l'une des revendications 1 à 4 caractérisé en ce que l'étape d'absorption (b) est réalisée de manière isotherme.

6.  Procédé selon la revendication 5 caractérisé en ce que l'étape d'absorption isotherme est réalisée dans une colonne refroidie par des échangeurs intégrés sur les plateaux de la colonne.

7.  Procédé selon la revendication 5 caractérisé en ce que l'étape d'absorption isotherme est réalisée dans une colonne où la phase liquide de chaque plateau est soutirée, refroidie dans un échangeur par un flux externe et réinjectée sur ledit plateau.

8.  Procédé selon l'une des revendications 1 à 7 caractérisé en ce que la phase gazeuse résiduelle issue de l'étape (c) est détendue dans une turbine de détente en produisant de l'énergie mécanique.

9.  Procédé selon l'une des revendications 1 à 8 caractérisé en ce que le gaz sortant de la turbine de détente est réchauffé de manière à récupérer de l'énergie frigorifique.

10. Procédé selon l'une des revendications 1 à 9, caractérisé en ce que le gaz utilisé à l'étape (b) provient d'une unité de craquage catalytique.

11. Procédé selon l'une des revendications 1 à 10, caractérisé en ce qu'on traite la phase liquide issue de l'étape (e) par un solvant sélectif non miscible à ladite phase et capable d'extraire les constituants aromatiques contenus dans ladite phase, qu'on recueille une phase dite raffinat exempte des constituants aromatiques et une phase dite extrait contenant le solvant et les constituants aromatiques, et que dans l'étape (f), la phase dite raffinat est mélangée à la fraction lourde B issue de l'étape (a) et que la phase dite extrait est fractionnée en d'une part le solvant qui est recyclé vers l'étape d'extraction et d'autre part les constituants aromatiques qui sont mélangés à l'essence hydrocarbonée entrant à l'étape (a).

**12.** Procédé selon l'une des revendications 10 ou 11, caractérisé en ce que l'étape d'extraction est une distillation réactive.

**13.** Procédé selon la revendication 10 ou 11, caractérisé en ce que l'étape d'extraction est une extraction liquide-liquide.

**14.** Procédé selon l'une des revendications précédentes, caractérisé en ce que l'essence hydrocarbonée entrant à l'étape (a) est constitué par un effluent de réformage catalytique.


## Patentansprüche

**1.** Verfahren zur Verminderung des Benzolgehaltes der kohlenwasserstoffhaltigen Benzine, dadurch gekennzeichnet, daß:
a) man fraktioniert wenigstens ein kohlenwasserstoffhaltiges Benzin in eine leichte mit Benzol angereicherte Fraktion A und eine schwere an Benzol verarmte Fraktion B,
b) man kontaktiert die leichte Fraktion A mit einem wenigstens einer Fraktion von Olefinen enthaltenden Gas, wobei deren Anzahl an Kohlenstoffatomen zwischen 2 und 5 pro Molekül liegt und diese Kontaktierung bei einer Temperatur zwischen
minus 40°C und 10°C und einem Druck zwischen 0,5 und 2 MPa vorgenommen wird,
c) man trennt am Austritt aus der gekühlten Absorbtionsstufe (b) ein an Olefinen verarmtes Restgas sowie eine flüssige Fraktion C ab, die an Olefinen angereichert ist,
d) man unterwirft diese flüssige Fraktion C einer Alkylierung bei einem Druck zwischen zwei und 10 MPa und bei einem Molverhältnis Benzol/olefin zwischen 0,5 und 2,
e) man fraktioniert das Gemisch aus der Stufe d) in eine gasförmige Phase, die zum grösseren Teil aus während eine flüssige Phase, die wenigstens zum Teil das nicht alkylierte Benzol und die Alkylbenzole enthält, und
f) man mischt die flüssige Phase aus der Stufe e) mit der schweren Fraktion B aus der Stufe a).

**2.** Verfahren nach Anspruch 1, dadurch gekennzeichnet, daß die flüssige Fraktion C ein Molverhältnis Benzol/Olefine zwischen 0,5 und 2 aufweist.

**3.** Verfahren nach einem der Ansprüche 1 - 2, dadurch gekennzeichnet, daß die Absorbtionsstufe (b) bei einem Druck kleiner als dem Druck der Alkylierungsstufe (d) durchgeführt wird.

**4.** Verfahren nach einem der Ansprüche 1 - 3, dadurch gekennzeichnet, daß die Alkylierungsstufe (d), mit einem Katalysator vom Typ entaluminierter Mordenit oder entaluminierter Faujasit durchgeführt wird.

**5.** Verfahren nach einem der Ansprüche 1 - 4, dadurch gekennzeichnet, daß die Absorbtionsstufe (b) in isotherm durchgeführt wird.

**6.** Verfahren nach Anspruch 5, dadurch gekennzeichnet, daß die isotherme Absorbtionsstufe in einer Kolonne durchgeführt wird, welche durch Wärmeaustauscher, die auf den Böden der Kolonne integriert sind, gekühlt wird.

**7.** Verfahren nach Anspruch 5, dadurch gekennzeichnet, daß die isotherme Absorbtionsstufe in einer Kolonne durchgeführt wird, wo die flüssige Phase jedes Bodens abgezogen, in einen Wärmeaustauscher durch einen äusseren Fluß gekühlt und auf diesen Boden wieder aufgegeben bzw. injiziert wird.

**8.** Verfahren nach einem der Ansprüche 1 - 7, dadurch gekennzeichnet, daß die gasförmige Restphase aus der Stufe c) in einer Entspannungsturbine entspannt wird, in der mechanische Energie erzeugt wird.

**9.** Verfahren nach einem der Ansprüche 1 - 8, dadurch gekennzeichnet, daß das aus der Entspannungsturbine austretende Gas derart erwärmt wird, daß Kälteenergie rückgewonnen wird.

**10.** Verfahren nach einem der Ansprüche 1 - 9, dadurch gekennzeichnet, daß das in Strafe (b) verwendete Gas aus einer katalytischen Krackeinheit stammt.

11. Verfahren nach einem der Ansprüche 1 - 10, dadurch gekennzeichnet, daß man die flüssige Phase aus der Stufe (e) mit einem selektiven Lösungsmittel behandelt, das mit dieser Phase nicht mischbar und in der Lage ist, die aromatischen in dieser Phase enthaltenen Bestandteile zu extrahieren, daß man eine sogenannte Raffinat-Phase frei von aromatischen Bestandteilen und eine sogenannte Extrakt-Phase, welche das Lösungsmittel und die aromatischen Bestandteile enthält, sammelt und daß in Stufe (f) die sogenannte Raffinat-Phase mit der schweren Fraktion B aus der Stufe (a) gemischt wird und daß die sogenannte Extrakt-Phase einerseits in das Lösungsmittel, das gegen die Extraktionsstufe rezyklisiert wird und andererseits die aromatischen Bestandteile fraktioniert wird, die mit dem in die Stufe (a) eintretenden kohlenwasserstoffhaltigen Benzin vermischt werden.

12. Verfahren nach einem der Ansprüche 10 oder 11, dadurch gekennzeichnet, daß die Extraktionsstufe eine reaktive Destillation ist.

13. Verfahren nach einem der Ansprüche 10 oder 11, dadurch gekennzeichnet, daß die Extraktionsstufe eine Flüssig-Flüssig-Extraktion ist.

14. Verfahren nach einem der vorhergehenden Ansprüche, dadurch gekennzeichnet, daß das kohlenwasserstoffhaltige in die Stufe (a) eintretende Benzin durch einen Abstrom der katalytischen Reformierung gebildet wird.

## Claims

1. A process for reducing the benzene content of hydrocarbon petrols, characterised in that:
   a) at least one hydrocarbon petrol is fractionated into a light fraction A with an increased benzene content and a heavy fraction B with a reduced benzene content,
   b) the light fraction A is contacted with a gas containing at least one fraction of olefins, in which the number of carbon atoms is between 2 and 5 per molecule, said contacting taking place at a temperature of between -40°C and 10°C and at a pressure of between 0.5 and 2MPa.
   c) at the end of the refrigerated absorption step (b), a residual gas with a reduced olefin content and a liquid fraction C with an increased olefin content are separated,
   d) said liquid fraction C is subjected to alkylation at a pressure of between 2 and 10MPa and with a benzene/olefin molar ratio of between 0.5 and 2,
   e) the mixture from step (d) is fractionated into a gaseous phase constituted in the main of gases which were not converted during alkylation, and into a liquid phase at least partly containing the non-alkylated benzene and alkylbenzenes.
   f) the liquid phase from step (e) is mixed with the heavy fraction B from step (a).

2. A process according to Claim 1, characterised in that the liquid fraction C has a benzene/olefin molar ratio of between 0.5 and 2.

3. A process according to one of Claims 1 to 2, characterised in that the absorption step (b) is carried out at a lower pressure than the pressure of the alkylation step (d).

4. A process according to one of Claims 1 to 3, characterised in that the alkylation step (d) is carried out with a catalyst of the dealuminated mordenite or dealuminated faujasite type.

5. A process according to one of Claims 1 to 4, characterised in that the absorption step (b) is carried out isothermally.

6. A process according to Claim 5, characterised in that the isothermal absorption step is carried out in a column cooled by exchangers which are integrated into the decks of the column.

7. A process according to Claim 5, characterised in that the isothermal absorption step is carried out in a column where the liquid phase on each deck is drawn off, cooled in an exchanger by an external flow and reinjected onto said deck.

8. A process according to one of Claims 1 to 7, characterised in that the residual gaseous phase from step (c) is brought to a lower pressure in a pressure reducing turbine, thereby producing mechanical energy.

9. A process according to one of Claims 1 to 8, characterised in that the gas issuing from the pressure reducing turbine is reheated in such a way as to recover refrigerating energy.

10. A process according to one of Claims 1 to 9, characterised in that the gas used in step (b) comes from a catalytic cracking unit.

11. A process according to one of Claims 1 to 10, characterised in that the liquid phase coming from step (e) is treated with a selective solvent which is non-miscible with said phase and which is capable of extracting the aromatic constituents contained in said phase, that a phase called a refined phase is collected which contains no aromatic constituents, and a phase called an extracted phase is collected which contains the solvent and the aromatic constituents, and that in step (f), the phase called the refined phase is mixed with the heavy fraction B coming from step (a) and the phase called the extracted phase is fractionated firstly into the solvent which is recycled to the extraction step and secondly into the aromatic constituents which are mixed with the hydrocarbon petrol which enters at step (a).

12. A process according to one of Claims 10 or 11, characterised in that the extraction step is a reactive distillation process.

13. A process according to Claim 10 or Claim 11, characterised in that the extraction step is a liquid-liquid extraction process.

14. A process according to one of the preceding claims, characterised in that the hydrocarbon petrol entering step (a) is constituted of an effluent from catalytic reforming.

FIG.1

Figure 2

EP 0 414 590 B1